# NEW EUROPEAN PATENT SPECIFICATION

(11) **EP 1 501 484 B2**
(45) Date of publication and mention of the opposition decision: **07.07.2010**
(45) Mention of the grant of the patent: 14.06.2006
(21) Application number: 02727807.6
(22) Date of filing: 29.04.2002
(51) Int. Cl.: A61K 9/16, A61K 9/20

(54) **A PROCESS FOR THE PREPARATION OF TABLETS FROM PHARMACEUTICALLY ACTIVE SUBSTANCES HAVING UNFAVOURABLE TABLETTING PROPERTIES WITH A GRANULATING LIQUID COMPRISING MICROCRYSTALLINE CELLULOSE**
HERSTELLUNG VON TABLETTEN VON PHARMAZEUTISCH AKTIVEN SUBSTANZEN MIT UNGÜNSTIGEN TABLETTIERUNGSEIGENSCHAFTEN MIT EINER MIKROKRISTALLINE ZELLULOSE ENTHALTENDEN GRANULIERFLÜSSIGKEIT
PROCEDE DE PREPARATION DE COMPRIMES A PARTIR DE SUBSTANCES, ACTIVES SUR LE PLAN PHARMACEUTIQUE, NE POSSEDANT PAS DE PROPRIETES FAVORABLES A LA MISE EN COMPRIME, AVEC UN LIQUIDE DE GRANULATION CONTENANT DE LA CELLULOSE MICROCRISTALLINE

(43) Date of publication of application: 02.02.2005
(73) Proprietor: EGIS Gyógyszergyár Nyrt, 1106 Budapest (HU)
(72) Inventor: FEKETE, Pál, H-1147 Budapest (HU); KIRALYNE IGNACZ, Mária, H-1161 Budapest (HU); SZLAVYNE SZELL, Zsuzsa, H-1106 Budapest (HU); GORA, Lászloné, H-2117 Isaszeg (HU); PALFI, Zoltánné, H-1172 Budapest (HU); JAMBOR, Istvánné, H-1182 Budapest (HU)
(74) Representative: Hirsch & Associés
(86) International application number: PCT/HU2002/000035
(87) International publication number: WO 2003/092658

(56) References cited:
- EP-A- 0 958 812
- EP-A1- 0 694 299
- WO-A-98/36737
- WO-A1-90/08542
- WO-A1-92/12633
- US- - 3 146 168
- VOIGT ET AL: "Pharmazeutische Technologie für Studium und Beruf" 1993 , PHARMAZEUTISCHE TECHNOLOGY FUER STUDIUM UND BERUF, XX, XX, PAGE(S) 208-213 XP002900619 page 208, paragraph 4 page 212, last paragraph
- 'Handbook of Pharmaceutical Excipients', 2000 article 'Cellulose, Microcrystalline'

## Description

### Field of the invention

The invention refers to a process for the preparation of tablets that can be well compressed and have good mechanical strength from pharmaceutically active ingredient(s) having unfavourable tabletting properties by admixing the pharmaceutically active ingredient(s) to conventional carriers used in tabletting, preferably binding agent(s) and/or filling agent(s) and/or disintegrating agent(s) and/or surfactant(s), granulating the mixture in the presence of a granulating liquid comprising microcrystalline cellulose by a kneading or a fluidization spraying process, and tabletting the granules obtained using further carriers conventionally employed in tabletting, preferably lubricant(s) and/or disintegrating agent(s) and/or glidant(s).

### Background of the invention

In the therapeutical use of pharmaceutical active ingredients, the most generally employed dosage form is the tablet (and coated tablet). The pharmaceutical firms manufacture a nearly inappreciable amount of tablet all over the world, taking into consideration that the tabletting capacity of a modem tabletting machine is about 500 000 pieces in every hour. In the same time, these tabletting machines of high performance need granules that can be tabletted without any problem since a huge damage may arise due to a short-fall of production when a machine stops or owing to the formation of an anormous amount of rejects when tablets of unsatisfactory quality are manufactured. Therefore, prior to tabletting, the active ingredients have to be transformed to granules that are suitable for tabletting. In general, such granules consist of particles having a size of 0.1-1.0 mm, and are prepared during different so-called preparatory procedures. A detailed description of these procedures can be found in the corresponding textbooks [such as Rácz, 1. and Selmeczi, B.: Gyógyszertechnológia (Pharmaceutical Technology), Vol. 1-3, Medicina, Budapest, 1994].

Principally, three kinds of preparatory process are employed:
- technology in which powders are mixed,
- dry granulation technology,
- wet granulation technology.

In case of the technology in which powders are mixed, the active ingredient is homogenized with carriers that can be easily tabletted. This technology is also called direct tabletting. The drawback of this process is that it can be employed only in a small part of the active ingredients that can be compressed also without carriers or are used in a low dose.

In case of the dry granulation technology, the active ingredient is homogenized with carriers that can be easily tabletted, the mixture obtained is compressed (formed to briquettes or pre-tabletted), the compressed material is ground, passed through a sieve, admixed to further carriers and tabletted. Again, this technology is employed only in a small part of the active ingredients - usually the ones that are sensitive to drying - since the tabletting properties of the active ingredients cannot be suitably improved by this method.

In case of the wet granulation technology, the active ingredient or, if desired, the mixture of the active ingredient and carriers used in tabletting is wetted with the so-called granulating agent. In this way, the surface of the solid particles will be covered by a film that forms from the materials dissolved in the granulating agent, and this film will be dried to the surface in the drying step that follows wetting. Thus, in the latter technology, the surface properties of the particles consisting of the active ingredient and the other solid carriers added to the active ingredient before granulation can be significantly - and, from the point of view of tabletting, favourably - changed. Recently, in the majority of cases, the wet granulation technology is employed as the preparatory process for tabletting.

In the wet granulation process, from the active ingredient(s) or, if desired, from a mixture of the active ingredient(s) and carriers which have a starting particle size of mainly lower than 0.1 mm, granules are prepared the 70 % by mass of which have a particle size of 0.1-1.0 mm. Although the wet granulation technology can be carried out in various equipments, recentty, the vortex flow or fluidization spraying granulation technology is exclusively used.

The granulation equipments contain mixer element vortex flow driven by two separate motors, The main mixer puts the material to be granulated in vortex motion, and kneads, aggregates the solid powders with the granulating liquid added into the equipment. The cutting head that rotates at high speed grinds the aggregates fromed during kneading. The resulting wet granules can be dried in a separate drier or in the granulation equipment itself.

In case of the fluidization spraying granulation technology, the particles to be granulated are in fluid state in the fluidization column owing to an air stream, and the granulating liquid is sprayed onto the particles. The wetted particles aggregate, and the granules can be dried in the apparatus when further granulating solution is not sprayed anymore.

Prior to granulation, various carriers are added to the active ingredient to obtain suitable tablet properties. Based on the role that influence the tablet properties, the carriers can be classified as follows,
- filling or diluting agents,
- binding agents,
- disintegrating agents that facilitate the dissolution of the active ingredient.

The filling or diluting agents are used, in case of the preparation of low dose tablets each containing lower than 100 mg of active ingredient, to increase the tablet mass, while in other cases to improve the tabletting properties of the active ingredient. Most often the following filling or diluting agents are employed: lactose monohydrate, mannitol, cellulose, microcrystalline cellulose, calcium hydrogen phosphate (anhydrous or dihydrate).

The binding agents are used to form a bond among the small particles of the active ingredients and carriers when preparing the granules on the one part, and to improve the mechanical strength of the final tablets on the other. The binding agents used in the wet granulation should dissolve in the granulating liquid. In general, the binding agents are high molecular natural or artificial materials, for example gelatin, maltodextrin, arabic gum, poly(vinyl pyrrolidone), cellulose ethers and esters (methyl cellulose, ethyl cellulose, hydroxypropyl methyl cellulose), acrylate copolymers, poly(vinyl acetate), poly(vinyl butyrate) etc. However, the bond between the particles can be also formed with the solutions of low molecular agents (such as lactose, mannitol, sucrose, glucose) or even with the active ingredients dissolved in the granulating liquid. In this case, the bond among the primary particles of the granules is provided by the active ingredient or carrier crystallizing among the particles.

The disintegrating agents and auxiliary agents that facilitate the dissolution of the active ingredient, respectively, are hydrophilic agents that swell in aqueous medium, thus, provide for the disintegration of the tablets to granules, and the disinregration of the granules to primary particles in aqueous medium, On the other hand, this class of carriers includes surfactants that facilitate wetting of the tablets and granules, and increase the solubility of the active ingredient. The most important disintegrating agents are the following: various starches (maize, potato, wheat), sodium or calcium salt of carboxymethyl starch, sodium or calcium salt of carboxymethyl cellulose, hydroxypropyl cellulose, poly(vinyl pyrrolidone) etc. It is to be noted that the starch and cellulose ethers used as disintegrating agent contain a lower amount of functional groups in comparison with the ones used as binding agent, thus, the starch and cellulose ethers suitable as disintegrating agent only swell in water, while the ones useful as binding agent form a colloidal solution in water.

In practice, the solution of the binding agent(s), low molecular carrier(s) or active ingredient(s) is used as the granulating liquid, however, also a solvent or solvent mixture can be used which dissolves a part or all of the components in the powder mixture to be granulated.

In addition to the carriers mentioned above, usually using a technology in which powders are mixed, also glidants and lubricants (anti-friction and anti-adhesion agents) are admixed to the granules prepared as described above to facilitate tabletting. The glidants promote the granules to fill the matrix slit of the tabletting machine more evenly, thus, reducing the mass deviation of the tablets. The anti-friction agents reduce the friction between the material to be tabletted or the final tablet and the matrix wall, while the anti-adhesion agents eliminate the adherence between the tabletting dies and the surface of the tablets and provide the bright surface of the tablets,

Most frequently, colloidal silica and talc are used as glidant, magnesium stearate, stearic acid an hydrogenized vegetable oils are employed as lubricant (anti-friction agent) and magnesium stearate and talc are used as anti-adhesion agent. In the development of the tabletting technology of a pharmaceutically active agent, the grade and amount of the above carriers as well as the granulation technology should be selected to obtain tablets that satisfy the quality requirements.

The quality of tablets are regulated essential by the pharmacopeial prescriptions. Although these prescriptions are not entirely unified yet, however, primarily those of the European (Ph- Eur) and US (USP) Pharmacopeias are considered as a standard for the international pharmaceutical industry.

At present, the most severe prescriptions referring to tablet parameters that are critical from the point of view of tabletting are as follows:

| Tablet parameter | Prescription of Ph.Eur. | Prescription of USP |
|---|---|---|
| Deviation of tablet mass | under 80 mg: ±10 % | none |
| | 80-250mg:± 7.5 % | |
| | above 250 mg: ± 5 % | |
| Active ingredient content of the tablet | nominal value ± 5 % | nominal value ±10 % |
| Deviation of active ingredient content | average value ± 15 % | nominal value ± 15 |
| | | relative deviation. not more than 1 % |
| Disintegration time | not more than 15 min. | it depends on the specific preparation |
| Dissolution of the active ingredient | there is no general requirement | 75-85 % in 30-60 min. |
| | | depending on the specific preparation |
| Friction loss | not more than 1 % | not more than 1% |
| Tablet strength^{x} | there is no requirement | there is no requirement |

| | | |
|---|---|---|
| ^{x} As to tablet strength, a value of 1 Mpa is advised for the tensile strength in the literature. (Tensile strength is the ratio of the collapsing force and the surface area that ruptures.) | | |

However, in case of several active ingredients, it is extremely difficult to attain the values prescribed for the critical tablet parameters because of the unfavourable tabletting properties of the active ingredients.

The following properties of the active ingredients influence the tablet manufacture unfavourably:
- strong adhesion,
- weak cohesion,
- bad solubility in water.
In case of strongly adhesive materials, partly high friction forces appear during compression which may cause the damage of the tablet when it is pressed out from the die, partly the tablets adhere to the surface of the die, thus, uneven tablet surface is obtained. In principle, the latter problem could be eliminated by the addition of a large amount of lubricant (anti-friction agent), however large amounts of lubricants (anti-friction agents) would reduce the tablet strength, prolong the disintegration of the tablets in aqueous medium (owing to the hydrophobic character of the anti-friction agents) and slow down the dissolution of the active ingredient, thus, the tablets obtained would not correspond to the quality prescriptions.

In case of materials having weak cohesion, the required tablet strength cannot be achieved by the compression. In principle, this problem could be eliminated by the addition of a large amount of binding agent, however, large amounts of binding agents would unacceptably prolong the disintegration of the tablets in aqueous medium and slow down the dissolution of the active ingredient, thus, again, the tablets obtained would not correspond to the quality prescriptions.

If the active ingredient has a bad solubility in water, the strong adhesion or weak cohesion thereof creates problem in an increased degree since the use of large amounts of both lubricants (anti-friction agents) and binding agents makes impossible to achieve the fast dissolution of the active ingredient

Because of the above difficulties, several patented processes have been developed for the preparation of the tablets of certain active ingredients having unfavourable tabletting properties.

In UK-P No. 1 445 983 the preparation of allopurinol tablets is described. To reduce the sizes of tablets containing 55-79 % by mass of active ingredient, 15-35 % by mass of indifferent filling agent (such as mannitol, dicalcium phosphate, microcrystalline cellulose or, preferably, lactose), 5-15 % by mass of disintegrating agent (such as alginic acid, sodium starch glycolate, guar gum, calcium carboxymethyl cellulose or, preferably, starch) and 1-10 % by mass of granulating agent (such as starch paste, gelatin, methyl cellulose or, preferably, poly(vinyl pyrrolidone) are employed.

In DE-P No. 342 774, the preparation of tablets containing 80-90 % by mass of allopurinol is described. The tablets comprise, in addition to the active ingredient, 5-8 % by mass of microcrystalline cellulose, 3.5-7 % by mass of a disintegration agent based on starch, furthermore binding agent (such as poly(vinyl pyrrolidone) and glidant (such as colloidal silica).

According to both documents mentioned above, the active ingredient is granulated as follows: the active ingredient, filling agent and disintegrating agent are mixed in powdered form, the mixture obtained is granulated using the aqueous solution of the binding agent [poly(vinyl pyrrolidone)], and, after drying and sieving, glidants are added followed by tabletting.

HU-P No. 191 384 describes the preparation of fast dissolution tablets containing at least 80 % by mass of alpha-methyldopa. 5-15 % by mass of microcrystalline cellulose as the filling agent, 1-3% by mass of sodium carboxymethyl cellulose as the disintegrating agent, furthermore a mixture of 0.5-5 % by mass of polyvinyl butyral and 0.5-5 % by mass of acrylate copolymer as binding agent are used in the process.

According to US-P No. 5,281,421, for the preparation of fast dissolution tablets containing gernfibrozil, 1-4 % by mass of a surface active agent having a HLB (hydrophilic lipophilic balance) value of 10-50 are used.

For the preparation of fast dissolution tablets containing gemfibrozil, HU-P 212 428 describes the use of 0.05-0.5 % by mass of diotilane [bis(2-ethyl-hexyl)-sodium-sulfosuccinate] as the surface active agent.

According to HU-P No. 196 710, ciprofloxacin tablets or capsules of fast dissolution can be prepared by using a dry binding agent based on microcrystalline cellulose, a disintegrating agent based on starch, a glidant, a further disintegrating agent based on a cellulose derivative and/or poly(vinyl pyrrolidone) and a lubricant. In the examples used for comparison in the description, the fast dissolution of ciprofloxacin is provided by the presence of disintegrating agents, maize starch and poly(virryl pyrrolidone).

In case of the last four documents, the granulation technology is performed by granulating the powder mixture of the active ingredient, binding agent(s) and optionally the disintegrating agent(s) with a solution of the binding agent (HU-P No. 191 384, US-P No. 5,281,421, HU-P No- 196 710) or only with the granulating solvent (HU-P No. 196 710).

Thus, up to now, the processes for improving the tabletting of pharmaceutically active ingredients have advised the use of more and more efficient binding agents and increasing the amount of the binding agents and the lubricants (anti-friction agents), respectively. In the same time, these steps have an unfavourable influence on the disintegration of the tablets and the dissolution rate of the active ingredient, respectively. To counteract this influence, the use of surface active agents is proposed. However, these may be also harmful since they can facilitate the absorption of toxic agents that are eventually present in the gastrointestinal system. Therefore, from the point of view of manufacturing pharmaceutical tablets, a process would be highly advantageous which would allow to use as low amount of binding agents (indispensable for the mechanical strength of the tablets) and lubricants (anti-friction agents) (essential for the compression) as possible and simultaneously improve the disintegration time of the tablets and the dissolution rate of the active ingredient.

When studying the tabletting technology of various pharmaceutically active ingredients, it has been found that even from active ingredients having unfavourable tabletting properties it is possible to produce tablets having good mechanical strength and to use only a low amount of high molecular binding agents and hydrophobic lubricants (anti-friction agents) thus, obtaining fast disintegrating tablets from which the active ingredient dissolves readily, when the granulation is carried out with a granulating liquid which is a suspension of 5-30 % of microcrystalline cellulose, 90 % of which have a particle size that is lower than 50 µm (the amount of the microcrystalline cellulose refers to the final tablet mass).

Several sorts of microcrystalline cellulose have been used in the tablet manufacture since about 1970 mainly as filling agent. At first, they have been employed primarily in case of direct compression technologies, however, at present, they are widely used also in wet granulation processes. For different purposes different grades of microcrystalline cellulose are commercially available which differ from each other in particle size, density and moisture (wet content) content [Wade. A. and Weller, P.J.: Handbook of Pharmaceutical Excipients, The Pharmaceutical Press, London, 1994].

The various grades of microcrystalline cellulose are distinguished by code numbers. Based on the code numbers, the characteristic physical parameters of each grade and the field of use advised by the manufacturers are given in the following table.

| Grade | Mean size in mm | Wet content in % | Density in g/cm³ | Field of use |
|---|---|---|---|---|
| 101 | 50 | max. 5 | about 0,30 | This type is used most frequently for wet granulation and direct tabletting or spheronization. |
| 102 | 100 | max. 5 | about 0.33 | Better flowability (gliding) properties than grade 101, mainly for direct compression. |
| 103 | . 50 | max. 3 | about 0,30 | Similar to grade 101, recommended for materials of lower moisture (wet) content which are sensitive to wetness. |
| 105 | 25 | max 5 | about 0.23 | Inert carrier for materials that do not crystallize. Sedimentation agent in dispersion and preparation of suppositories. |
| 112 | 100 | max. 3 | about 0,33 | Similar to grade 102. recommended for materials of lower moisture (wet) content which are sensitive to wetness. |
| 200 | 180 | max. 5 | about 0,38 | This grade has the best flowability (gliding) properties, it is recommended for direct tabletting. |
| 301 | 50 | max. 5 | about 040 | This grade is similar to grade 101 but has higher density and better flowability (gliding) properties, it is recommended for direct tabletting. |
| 302 | 100 | max. 5 | about 40 | This grade is similar to grade 102 but has higher density and better flowability (gliding) properties, it is recommended for direct tabletting. |
| 12 | 160 | max.5 | about 0.39 | Good flowability (gliding) properties, recommended for direct tabletting. |
| 20 | 30 | max. 5 | about 0.21 | Similar quality and use than those of grade 105. |

According to the state of art, the different grades of microcrystalline cellulose described above are used by admixing the microcrystalline cellulose to the active ingredient and, if desired, other carriers in powdered form, and, in case of direct tabletting, the powder mixture is tabletted. If the flowability (gliding) and/or compressing properties of the powder mixture is not sufficient, wet granulation is carried out. In the latter procedure, the powder mixture is granulated with the solution of a binding agent and/or with a suitable solvent, the granules obtained are dried, sieved, mixed with a glidant and tabletted.

Thus, the possible use of microcrystalline cellulose in aqueous or aqueous alcoholic suspensions for improving the tabletting technologies of active ingredients having unfavourable tabletting properties has not been known in the prior art.

### Summary of the invention

Thus, according to the invention, tablets that can be well compressed and have good mechanical strength are prepared from pharmaceutically active ingradient(s) having unfavourable tabletting properties wherein the active ingredient is deramciclane or a pharmaceutical acceptable acid addition salt therof by admixing the pharmaceutically active ingredient(s) to conventional carriers used in tabletting, preferably binding agent(s) and/or filling agent(s) and/or disintegrating agent(s) and/or surfactant(s), granulating the mixture in the presence of a granulating liquid by a kneading or a fluidization spraying process, and tabletting the granules obtained using further carriers conventionally employed in tabletting, preferably lubricant(s) and/or disintegrating agent(s) and/or glidant(s), in which process the granulating liquid is a suspension of 5-30 % of microaystalline cellulose - 90 % of which have a particle size that is lower than 50 µm, and the amount of the microcrystalline cellulose refers to the final tablet mass - in water and/or ethanol and/or isopropanol, and said suspension may contain also a part of the ingredients of the granules in dissolved form.

### Description of the preferred embodiments

In the preparation of the granules, conventional carriers that are used in tabletting are employed. Preferably, such carriers include one or more binding agent(s) (the total amount thereof is 1-1 D % of the final tablet mass, in general), optionally one or more filling agent(s) (if used, in general, the total amount thereof is not higher than 50 % of the final tablet mass, however, depending on the active ingredient content, the total amount thereof can be also 99 %), one or more disintegrating agent(s) (the total amount thereof is 1-40 % of the final tablet mass, in general) and optionally one or more surfactant(s) (if used, the total amount thereof is not higher than 5 % of the final tablet mass, in general).

For tabletting the final granules, in general, further carriers conventionally used in tabletting are employed. Such carriers include, preferably, one or more lubricant(s) (the total amount thereof is 0.1-5 % of the final tablet mass, in general), optionally one or more disintegrating agent (if used, the total amount thereof is not higher than 20 % of the final tablet mass, in general), and optionally one or more glidant(s) (if used, the total amount thereof is not higher than 3 % of the final tablet mass, in general).

In the process of the invention, the microcrystalline cellulose content of the final tablet is wholly or partially suspended in the granulating liquid and this suspension is used for the granulation. The granulation liquid can be water, ethanol, isopropanol or any mixtures thereof or a solution of the binding agents or any other material(s) that are present in the final tablet in water, ethanol, isopropanol or any mixtures thereof. For the preparation of the suspension, only microcrystalline celluloses having low particle size that is 90 % of cellulose particles are smaller than 50 µm (such as grades 20, 105, 101, 103), preferably 90 % of the cellulose particles are smaller than 25 µm (such as grades 20, 105) are employed since microcrystalline celluloses of higher particle size or higher density cannot be suspended suitably.

In the preparation of the suspension of the microcrystalline cellulose, the binding agents to be dissolved in the solvent or solvent mixture include binding agents generally used in tablet preparation or filling agents. For example, hydrophilic polymers such as poly(vinyl pyrrolidone) or vinylpyrrolidone/vinylacetate copolymer, poly(vinyl alcohol), poly(ethylene glycol), cellulose ethers such as ethyl cellulose, hydroxyethyl cellulose, hydroxypropyl methyl cellulose, hydroxypropyl cellulose, sodium carboxymethyl cellulose, starch hydrolysates such as maltodextrin, protein hydrolysates such as gelatin etc. can be dissolved. Of the filling agents, sugar and sugar alcohols such as lactose, glucose, sucrose, mannitol, sorbitol etc. can be dissolved, primarily.

Thus, according to the process of the invention, the microcrystalline cellulose content of the tablet to be prepared is wholly or partially suspended in water and/or ethanol and/or isopropanol. In the thus-obtained granulating liquid, a part of the components of the granules, generally 3D % by mass thereof at the most, can be also dissolved. These components are dissolved either one by one, in any order of sequence or simultaneously. It is also possible to dissolve the active ingredient or a part thereof in the granulating liquid.

The composition of the granulating suspension is suitably adjusted to obtain a liquid that can be still poured, in case of vortex flow granulation, or satisfactorily sprayed, in case of fluidization spraying granulation, similarly to the granulating solvents. As to the concentration of the binding agents, experts of tablet manufacture have the corresponding experience. The amount of the microcrystalline cellulose is 5-30 % of the total mass of the granulating liquid.

Using the granulating liquid of the invention, the granulation process itself and from the granules obtained the tabletting are carried out in a manner known per se.

The process of the invention i.e- the novel use of microcrystalline cellulose according to the invention is a significant progress in the field of granulation technologies since the microcrystalline cellulose particles applied to the surface of the particles of the materials to be granulated from the suspension form a layer thereon thereby improving the compressing and lubricaton (friction) properties of the particles. Consequently, the quantity of binding agents and glidants can be reduced in the composition and suitable tablet strength as well as fast dissolution of the active ingredient are obtained.

Thus, the use of the process of the invention has to be taken into consideration primarily for deramciclane [N,N-dimethyl-2-[(1R,2S,4R)-2-phenyl-2-bomyloxy]ethylamine] that "stick S" in tabletting or the dissolution rate of which is significantly reduced by the polymeric binding agents employed. In tabletting the granules thereof prepared by the conventional granulation process, sticking problems on the surface of the tablet as well as "drawing problems" on the side of the tablet are experienced, regularly. In case of using the binding agents in an amount that is needed to produce the required tablet strength, the dissolution of the latter active ingredients is very slow.

The prepared tablets have high breaking strength preferably above 90 N, and disintegration time, preferably 5-9 min.

The invention is further elucidated by means of the following

### Examples.

### Example 1

### Preparation of tablets containing deramciclane

To prepare the granulating liquid. 45 g of hydroxypropyl methyl cellulose are dissolved in 900 ml of water, and, in the solution obtained, 48 g of microcrystalline cellulose grade 105 (90 % of which have a particle size that is lower than 25 µm) are dispersed.

To prepare the granules, 126 g of deramciclane fumarate, 180 g of mannitol and 300 g of microcrystalline cellulose grade 101 (90 % of which have a particle size that is lower than 50 µm) are transfered into the container of a fluidization granulating apparatus type Glatt GPCG 1, fluidized by introducing air at 40 °C, and the above granulating liquid is sprayed on the fluidized powder in about 20 minutes. The particles obtained are dried, and passed through a sieve having a mesh size of 1 mm. To the granules obtained, 54 g of sodium carboxymethyl cellulose (disintegrating agent), 18 g of talc (lubricant) and 9 g of magnesium stearate (lubricant) are added, and the mixture is tabletted to produce lentiform tablets of 9 mm diameter. Each tablet has a mass of 260 mg. During tabletting no signs indicating sticking were found either on the dies, or on the surface of the tablets.

The critical parameters of the tablets determined according to the relevant prescriptions of the European Pharmacopeia are as follows:

| Compressing force | Wearin | Disinteg ration | Breaking strength in | Height in | Mass | Dissolution in %** after | | |
|---|---|---|---|---|---|---|---|---|
| in kN | % | in min | N | mm | in mg | 5 min | 15 min | 30 min |
| 8 | 0.23 | 3.97 | 66.7 | 4.35 | 260 | | | |
| 10 | 0.19 | 8.6 | 61.24 | 4.23 | 260 | | | |
| 12 | 0.19 | 7.33 | 93.31 | 4.18 | 262 | | | |
| 14 | 0.18 | 8.4 | 97.74* | 4.16 | 283.1 | 41.65 | 86.15 | 92.34 |
| 16 | 0.19 | 8.55 | 106.5 | 4.13 | 265.5 | | | |
| 20 | 0.26 | 9.31 | 110.17 | 4.10 | 265.9 | | | |

| | | | | | | | | |
|---|---|---|---|---|---|---|---|---|
| ^{x} The value of tensile strength: 2.61 MPa. | | | | | | | | |
| ^{xx} The dissolution rate is not prescribed in the European Pharmacopeia. | | | | | | | | |

Taking into consideration the requirements given on page 8, the quality of the deramciclane tablets prepared is very favourable.

### Example for comparison

For comparison, the deramciclane tablets were prepared by using a granulating liquid that did not contain any microcrystalline cellulose, while the amount of microcrystalline cellulose in the powder mixture was increased correspondingly. In tabletting, the surface of the lower press die became dull indicating the adherence of a thin layer, thus, the sticking of the composition.

The critical parameters of the tablets determined according to the relevant prescriptions of the European Pharmacopeia are as follows:

| Compres sing force | Wearin | Disinteg ration | Breaking strength In | Height in | Mass | Dissolution in %** after | | |
|---|---|---|---|---|---|---|---|---|
| in kN | % | in min | N | mm | in mg | 5 min | 15 min | 30 min |
| B | 0.19 | 1.55 | 46.7 | 4.50 | 264.9 | | | |
| 10 | 0.15 | 2.30 | 59.4 | 4-33 | 263.6 | | | |
| 12 | 0.23 | 2.82 | 67.0 | 4.25 | 263.4 | | | |
| 14 | 0.19 | 3.53 | 74,6* | 4.22 | 265.4 | 73.71 | 95.89 | 98.79 |
| 16 | 0.19 | 3.73 | 79.5 | 4.13 | 261.0 | | | |
| 20 | 0.27 | 4.10 | 86.4 | 4.10 | 262.8 | | | |

| | | | | | | | | |
|---|---|---|---|---|---|---|---|---|
| * The value of tensile strength: 1.96 MPa. | | | | | | | | |
| ^{xx} The dissolution rate is not prescribed in the European Pharmacopeia. | | | | | | | | |

As to the physical parameters of the tablets obtained, the breaking strength and disintegration time are lower, and, correspondingly, the dissolution rate is somewhat higher than those of tablets obtained in Example 1. However, the appearance of the tablets is not suitable owing to the sticking to the dies.

### Example 2 (not part of the invention)

### Preparation of tablets containing hydrochlorothiazide

1.5 kg of lactose are dissolved in 6000 ml of water warmed to 60-70 °C, and, in the solution obtained, 1.5 kg of microcrystalline cellulose grade 105 are suspended. During use, the suspension is maintained at 60-70 °C. 7.5 kg of hydrochlorothiazide are fluidized in a fluidization granulating apparatus type Aeromatic STE 15 using air stream at 60±5 °C, and the granulating liquid prepared above is sprayed on the active ingredient at 200 rietlmin feeding (feeding velocity) and under 1.5 bar spraying pressure. Then the granules are dried until the wet content is not higher than 1 % by mass, and passed through a sieve having a mesh size of 1 mm.

The granules obtained can be diluted with carriers conventional used in direct tabletting and/or with active ingredients having blood pressure lowering affect or the granules thereof, then compressed to obtain tablets containing one or more active ingredients.

To prepare a combination composition wherein each tablet contains 15 mg of hydrochlorothiazide and 50 mg of captopril [(S)-9-(3-mercapto-2-methyl-1-oxopropyl)-L-proline], 1.05 kg of the above granules, furthermore 2.50 kg of captopril, 4.85 kg of spray dried lactose monohydrate, 4.85 kg of microcrystalline cellulose grade 102, 1.50 kg of maize starch, 0.15 kg of stearine and 0.05 kg of magnesium stearate are transferred into a gravity mixer of 50 litre capacity, homogenized for 25 minutes, then compressed to 300 mg tablets by a rotary tabletting machine Manesty Betapress using flat flanged press dies of 10 mm diameter. Each tablet obtained contains 15 mg of hydrochlorothiazide and 50 mg of captopril.

To prepare a combination composition wherein each tablet contains 25 mg of hydrochlorothiazide and 25 mg of captopril, 1,750 kg of the above granules, furthermore 9.250 kg of captopril, 2.425 kg of spray dried lactose monohydrate, 2.425 kg of microcrystalline cellulose grade 102, 0.750 kg of maize starch, 0.075 kg of stearine and 0.025 kg of magnesium stearate are transfered into into a gravity mixer of 30 litre capacity, homogenized for 25 minutes; then compressed to 174 mg tablets by a rotary tabletting machine Manesty Betapress using flat flanged press dies of 8 mm diameter or lentiform and engraved or divided dies. Each tablet obtained contains 25 mg of hydrochlorothiazide and 25 mg of captopril.

3 further batches were manufactured from each latter tablet composition, and the critical parameters of the tablets obtained were as follows- The tablet parameters were determined according to the relevant prescriptions of the US Pharmacopeia.

Requirement for dissolution of the USP:
dissolution of the active ingredients using 6 tablets for the determination:

| | |
|---|---|
| captopril after 20 min | not lower than 85 %, |
| hydrochlorothiazide after 20 min | not lower than 65 %. |

| Captopril/hydrochlorothiazide tablet 50/15 mg | Requirement | Batch No. 1 | Batch No. 2 | Batch No. 3 |
|---|---|---|---|---|
| Average mass in g | 0.284-0.314 | 0.3096 | 0.3062 | 0.3034 |
| Height in mm | 3.10-3.50 | 3.30 | 3.32 | 3.25 |
| Mass deviation in % | ±5 | 2.2; +3.3 | -1.4 ; +2.0 | -2.0; +3.4 |
| Breaking strength in N | min. 35 | 51 | 43 | 48 |
| Tensile strength in Mpa | min. 1 | 1.54 | 1.29 | 1.47 |
| Wear loss in % | 1 | 0.01 | 0.06 | 0.03 |
| Dissolution of the active ingredient in % | | 103.0-106.1 | 104.4-106.7 | 96.8-101.4 |
| captopril after 10 min | | | | |
| hydrochlorothlazide after 10 min | | 97.1-96.4 | 88.7-93.0 | 75.0-87.0 |

| Captopril/hydrochlorothiazide tablet 25/25 mg | Requirement | Batch No.1 | Batch No. 2 | Batch No. 3 |
|---|---|---|---|---|
| Average mass in g | 0.161-0.1871 | 0.1757 | 0.1750 | 0.1766 |
| Height in mm | 2.73-3.07 | 296 | 294 | 2.93 |
| Mass deviation in % | ±5 | -1.0; +2.8 | -4.6 ; +2.3 | -2.7; +2.0 |
| Breaking strength in N | min. 30. | 33 | 36 | 42 |
| Tensile strength in MPa | min.1 | 1.39 | 1.53 | 2.04 |
| Wear loss in % | max. 1 | 0.04 | 0.03 | 0.07 |
| Dissolution of the active ingredient in % | | 101.7 105.3 | 102.3-106.4 | 103.7-107.2 |
| captopril after 10 min | | | | |
| hydrochlorothiazide after 10 min | | 94.7-100.3 | 99.5-105.3 | 102.1-107.5 |

Thus, according to the test results, tablets having good mechanical strength could be produced. From the tablets obtained, the active ingredients could be dissolved significantly faster than prescribed by the US Pharmacopeia.

### Example 3 (not part of the invention)

### Preparation of tablets containing ranitidine

1.05 kg of microcrystalline cellulose grade 105 are suspended in a mixture of 7000 ml of 95 % by volume of ethanol and 1000 ml of water. The materials to be granulated (11.76 kg of ranitidine hydrochloride and 3.08 kg of microcrystalline cellulose grade 105) are homogenized in a vortex flow granulation equipment Diosna of 100 litre capacity for about 3 minutes. The above granulating suspension is added in about 5 minutes under constant mixing (stirring) and the granulation procedure is carried out for further 12 minutes. The wet particles are dried in a fluidization granulating apparatus type Aeromatic STE 15, the dry granules are regranulated by passing them through a sieve having a mesh size of 0.8 mm, and the granules obtained are homogenized with 3.50 kg of microcrystalline cellulose grade 102, 1.05 kg of sodium carboxymethyl cellulose and a mixture of 0.14 kg of magnesium stearate and 0.07 kg of silica in a gravity mixer of 100 litre capacity. From the homogenized mixture, 295 mg lentiform tablets of 10 mm diameter are prepared using a rotary tabletting machine Manesty Betapress or 590 mg oviform tablets of 17.5 mm length and 7.5 mm width are produced by a tabletting machine Kilian RTS 21. The tablets obtained contain 150 mg and 300 mg of ranitidine, respectively.

The critical parameters of the tablets obtained were as follows. The tablet parameters were determined according to the relevant prescriptions of the US Pharmacopeia.

Requirement for dissolution of the USP:
dissolution of the active ingredient using 6 tablets for the determination:
in 45 minutes not less than 85 %.

| Ranitidine tablet | 150 mg | 150 mg | 300 mg | 300 mg |
|---|---|---|---|---|
| Parameter | Requirement | Value determined | Requirement | Value determined |
| Average mass in g | 0.2803-0-3097 | 0.2934 | 0.6605 - 0.6195 | 0.5870 |
| Mass deviation in % | ±5 | -2.1;+2.5 | -1.9;+1.5 | -2.0; +3.4 |
| Height in mm | 4.14-4.66 | 4.50 | 4.79-5.41 | 5.04 |
| Breaking strength in N | min.60N | 105 | min. 100 N | 159 |
| Tensile strength in Mpa | min.1 | 2.33 | min. 1 | 1.80 |
| Wear loss in % | max. 1 | 0.0 | max. 1 | 0.0 |

The tablets were coated with a film based on 12 mg and 24 mg of water-soluble hydroxypropyl methyl cellulose, respectively, then the dissolution rate was determined according to the prescription of the USP. The following values were Obtained;

| Dissolution of the active ingredient in | in case of the 150 mg tablet in % by weight | in case of the 300 mg tablet In % by weight |
|---|---|---|
| 15 min. | 85.5-90.0 | 76.0-85.4 |
| 30 min. | 92.9-99.6 | 94.5-100.9 |

Thus, based on the test data, tablets having very good mechanical strength could be prepared from which the dissolution of the active ingredient is significantly faster than prescribed by the USP.

### Example 4 (not part of the invention)

### Preparation of tablets containing metoprolol

3-2 kg of poly(vinyl pyrrolidone) are dissolved in a mixture of 17 litre of water and 17 litre of 95 % by volume of ethanol, and, in the solution obtained, 4.8 kg of microcrystalline cellulose grade 105 are suspended. The materials to be granulated (40.0 kg of metoprolol tartrate, 61.60 kg of microcrystalline cellulose grade 105, 12.0 kg of sodium carboxymethyl starch, 1.6 kg of silica) are homogenized in vortex flow granulation equipment Diosne of 400 litre capacity for about 3 minutes. The above granulating suspension is added in about 1-2 minutes under constant mixing (stirring), and the granulation procedure is carried out for further 10 minutes. The wet particles are dried in a fluidization granulating apparatus type Glati WSG 120, the dry granules are regranulated by passing them through a sieve having a mesh size of 0.8 mm, and the granules obtained are homogenized with a mixture of 3.2 kg of magnesium stearate and 1.6 kg of silica in a gravity mixer of 450 litre capacity.

From the homogeneous mixture 80, 160 and 320 mg tablets can be compressed on a rotary tabletting machine (e.g. Manesty Betapress, Kilian T300 A100, Fette Perfecta 300 etc.) using flat flanged or lentiform and engraved or divided dies of 6 mm, 8 mm and 10 mm diameter. The above tablets contain 25 mg, 50 mg and 100 mg of metoprolol tartrate, respectively.

3 further batches were manufactured from each latter tablet composition, and the critical parameters of the tablets obtained were as follows. The tablet parameters were determined according to the relevant prescriptions of the US Pharmacopeia.

Requirement for dissolution of the USP:
dissolution of the active ingredient using 6 tablets for the determination:

| | |
|---|---|
| after 30 min | not lower than 80 %. |

| Metoprolol tablet 25 mg | Requirement | Batch No. 1 | Batch No. 2 | Batch No. 3 |
|---|---|---|---|---|
| Average mass | in g 0,072-0.088 | 0.0826 | 0.0609 | 0.0812 |
| Mass deviation in % | ± 10 | -4.6; +5.3 | -2,1;+3.4 | -1.2; +1.7 |
| Height in mm | 2.71-3.19 | 2.85 | 2.87 | 2.86 |
| Breaking strength in N | min. 20 | 35 | 36 | 38 |
| Tensile strength in Mpa | min. 1 | 2.04 | 2.09 | 2.21 |
| Wear loss in % | max 1 max.1 | 0.11 0.11 | 0,12 0.12 | 0.11 0.11 |
| Dissolution of the active ingredient in % | | 98.2-104.4 | 94.8-102.3 | 96.1-100.5 |
| after 10 min | | | | |

| Metoprolol tablet 50 mg | Requirement | Batch No.1 | Batch No. 2 | Batch No. 3 |
|---|---|---|---|---|
| Average mass in g | 0.148-0.172 | 0.1605 | 0.1605 | 0.1597 |
| Mass deviation in % | ± 10 | -2.5; +3.3 | -2.5 +1.6 | -1.7; +3.2 |
| Height in mm | 3.10-3.50 | 3.27 | 3.24 | 3.25 |
| Breaking strength in N | min. 30 | 40 | 40 | 46 |
| Tensile strength in Mpa | min. 1 | 1.52 | 1.54 | 1.76 |
| Wear loss in % | max.1 | 0.00 | 0.00 | 0.00 |
| Dissolution of the active ingredient in % | | 91.7-107.1 | 95.5-101.3 | 88.7-90.2 |
| After 10 min | | | | |

| Metoprolol tablet 100 mg | Requirement | Batch No.1 | Batch No. 2 | Batch No. 3 |
|---|---|---|---|---|
| Average mass in g | 0.304-0.336 | 0.3242 | 0.321 | 0.3233 |
| Mass deviation In % | ±10 | -2.1; +2.2 | -1.7 ; +22 | 3.5; +3.0 |
| Height in mm | 3.95-4.45 | 4.37 | 4.27 | 4.28 |
| Breaking strength in N | min. 40 | 44 | 45 | 51 |
| Tensile strength in MPa | min. 1 | 1.01 | 1.05 | 1.19 |
| Wear loss in % | max. 1.0 | 0.03 | 0.06 | 0.06 |
| Dissolution of the active ingredient in % | | 91.6-97.6 | 69.35-91.93 | 95.0-101.1 |
| after 10 min | | | | |

Thus, based on the test data, tablets having very good mechanical strength could be prepared from which the dissolution of the active,ingredient is significantly faster than prescribed by the USP.

### Example 5

### Preparation of tablets containing deramciclane

To prepare the granulating liquid, 192 g of poly(vinyl pyrrolidone) are dissolved in 720 ml of water, and, in the solution obtained, 120 g of microcrystalline cellulose grade 105 (90 % of which have a particle size that is lower than 25 µm) are dispersed.

To prepare the granules, 504 g of deramcidane fumarate, 360 g of mannitol and 480 g of microcrystalline cellulose grade 101 (90 % of which have a particle size that is lower than 50 µm) are transfered into the container of a fluidization granulating apparatus type Glatt GPCG 1, fluidized by introducing air at 40 °C, and the above granulating liquid is sprayed on the fluidized powder in about 23 minutes. The particles obtained are dried, and passed through a sieve having a mesh size of 1 mm. To the granules obtained, 120 g of sodium carboxymethyl cellulose (disintegrating agent) and 24 g of magnesium stearate are added, and the mixture is tabletted to produce lentiform tablets of 7 mm diameter using a Manesty Betapress tabletting machine. Each tablet has a mass of 150 mg ± 5 % and contains 30 mg of deramciclana base. During tabletting no signs indicating sticking were found either on the dies, or on the surface of the tablets.

The critical parameters of the tablets determined according to the relevant prescriptions of the European Pharmacopeia are as follows:

| Compressing force | Wearin | Disintegration | Breaking strength in | Height in | Mass | Dissolution in %* after | | |
|---|---|---|---|---|---|---|---|---|
| In kN | % | in min | N | mm | in mg | 5 min | 15 min | 30 min |
| 6 | 0.08 | 8.2 | 39.5 | 4.48 | 148,7 | | | |
| 8 | 0.10 | 9.1 | 49.5 | 4.38 | 148.3 | | | |
| 10 | 0.05 | 9.3 | 53.6 | 4.36 | 148.0 | | | |
| 11 | 0.09 | 9.2 | 53.9 | 4.34 | 148.9 | 39.7 | 82.6 | 95.6 |
| 12 | 0.02 | 9.6 | 55.5 | 4.40 | 151.5 | | | |

Dissolution test was carried out in 900 ml of buffer having a pH value of 6.8 and using a bladed device at 50 revolution/min.

Taking into consideration the requirements given on page 8, the quality of the deramciclane tablets prepared is very favourable.

### Example 6

### Preparation of tablets containing deramciclane

To prepare the granulating liquid, 192 g of poly(vinyl pyrrolidone) are dissolved in 700 ml of water, and, in the solution obtained, 120 g of microcrystalline cellulose grade 105 (90 % of which have a particle size that is lower than 25 µm) are dispersed.

To prepare the granules, 504 g of deramciclane fumarate, 360 g of mannitol and 480 g of microcrystalline cellulose grade 101 (90 % of which have a particle size that is lower than 50 µm) are transfered into the container of a fluidization granulating apparatus type Glatt GPCG 1, fluidized by introducing air at 40 °C, and the above granulating liquid is sprayed on the fluidized powder in about 20 minutes. The particles obtained are dried, and passed through a sieve having a mesh size of 1 mm. To the granules obtained, 120 g of sodium carboxymethyl cellulose (disintegrating agent), 120 g of microcrystalline cellulose grade 102 (90 % of which have a particle size that is lower than 90 µm) and 36 g of magnesium stearate are added, and the mixture is tabletted to produce lentiform tablets of 10 mm diameter using a Manesty B3B tabletting machine. Each tablet has a mass of 320 mg and contains 60 mg of deramciclane base. During tabletting no signs indicating sticking were found either on the dies, or on the surface of the tablets.

## Claims

1. A process for the preparation of tablets that can be well compressed and have good mechanical strength from pharmaceutical active ingredient(s) having unfavourable tabletting properties, wherein the active ingredient is deramciclane or a pharmaceutical acceptable acid addition salt thereof, by:
- admixing the pharmaceutically active ingredient(s) to conventional carriers used in tabletting, preferably binding agent(s) and/or filling agent(s) and/or disintegrating agent(s) and/or surfactant(s),
- granulating the mixture in the presence of a granulating liquid by a kneading or a fluidization spraying process, and
- tabletting the granules obtained using further carriers conventionally employed in tabletting, preferably lubricant (s) and/or disintegrating agent (s) and/or glident (s), in which the granulating liquid is a suspension of 5-30 % of microcrystalline cellulose - 90 % of which have a particle size that is lower than 50µm, and the amount of the microcrystalline cellulose refers to the final tablet mass - in water and/or ethanol and/or isopropanol, and said suspension may contain also a part of the ingredients of the granules in dissolved form.

2. A process as claimed in Claim 1, in which 90 % of the microcrystalline cellulose suspended in the granulating liquid have a particle size that is lower than 25 µm.

3. A process as claimed in Claim 1 or 2, in which the granulating liquid contains-in addition to the microcrystalline cellulose - poly(vinyl pyrrolidone), hydroxypropyl methyl cellulose, hydroxypropyl cellulose, ethyl cellulose and/or gelatin as binding agent in dissolved form.

4. A process as claimed in Claim 1 or 2, in which the granulating liquid contains - in addition to the microcrystalline cellulose - lactose, mannitol and/or glucose as filling agent in dissolved form.

5. Tablets as prepared by the process claimed in Claim 1 having high breaking strength and disintegration time.

6. Tablets as claimed in Claim 5 having a breaking strength of above 90 N.

7. Tablets as claimed in Claim 5 having a disintegration time of 5-9 minutes.

## Patentansprüche

1. Verfahren für die Herstellung von Tabletten, die gut komprimiert werden können und gute mechanische Festigkeit aufweisen, aus einem oder mehreren pharmazeutischen Wirkstoff(en) mit ungünstigen Tablettiereigenschaften, wobei der Wirkstoff Deramciclan oder ein pharmazeutisch annehmbares Säureadditionssalz davon ist, durch:
- Beimischen des oder der pharmazeutischen Wirkstoffe(s) zu herkömmlichen Trägem, die in der Tablettierung verwendet werden, die vorzugsweise ein oder mehrere Bindemittel und/oder Füllmittel und/oder Desintegrationsmittel und/oder oberflächenaktive(s) Mittel sind,
- Granulieren des Gemisches in Gegenwart einer Granulierungsflüssigkeit durch ein Knet- oder einen Fluidisierungssprühprozess, und
- Tablettieren der erhaltenen Granula unter Verwendung weiterer Träger, die für gewöhnlich in der Tablettierung verwendet werden, die vorzugsweise ein oder mehrere Schmiermittel und/oder Desintegrationsmittel und/oder Fließregulierungsmittel sind, wobei die Granulierungsflüssigkeit eine Suspension aus 5 bis 30% mikrokristalliner Zellulose - wovon 90% eine Partikelgröße kleiner als 50 µm aufweisen und wobei sich die Menge der mikrokristallinen Zellulose auf die endgültige Tablettenmasse bezieht - in Wasser und/oder Ethanol und/oder Isopropanol ist und die Suspension auch einen Teil der Inhaltsstoffe der Granula in gelöster Form enthalten kann.

2. Verfahren nach Anspruch 1, wobei 90% der mikrokristallinen Zellulose, die in der Granulierungsflüssigkeit suspendiert ist, eine Partikelgröße aufweisen, die kleiner als 25 µm ist.

3. Verfahren nach Anspruch 1 oder 2, wobei die Granulierungsflüssigkeit - zusätzlich zu der mikrokristallinen Zellulose - Poly(vinylpyrrolidon), Hydroxypropylmethylzellulose, Hydroxypropylzellulose, Ethylzellulose und/oder Gelatine als Bindemittel in gelöster Form enthält.

4. Verfahren nach Anspruch 1 oder 2, wobei die Granulierungsflüssigkeit - zusätzlich zu der mikrokristallinen Zellulose - Lactose, Mannitol und/oder Glucose als Füllmittel in gelöster Form enthält.

5. Tabletten, die durch das Verfahren in Anspruch 1 hergestellt sind, mit hoher Bruchfestigkeit und Desintegrationszeit.

6. Tabletten nach Anspruch 5, mit einer Bruchfestigkeit von über 90 N.

7. Tabletten nach Anspruch 5, mit einer Desintegrationszeit von 5 bis 9 Minuten.

## Revendications

1. Un procédé de préparation de comprimés convenablement pressés et présentant une bonne résistance mécanique, à partir d'ingrédients actifs du point de vue pharmaceutique présentant des propriétés défavorable de misse sous forme de comprimés, où l'ingrédient actif est la deramciclane ou un sel d'addition d'acide acceptable du point de vue pharmaceutique en dérivant, consistant:
- à mélanger l'ingrédient utile du point de vue pharmaceutique à des supports classiques utilisés dans la formation du comprimé, de préférence des agents de liaison et/ou des agents de charge et/ou des agents de désintégration et/ ou des agents tensio-actifs,
- à granuler le mélange en présence d'un liquide de granulation par un procédé de pulvérisation par fluidisation ou par malaxage et
- à mettre des granulés obtenus sous forme de comprimés en utilisant des supports supplémentaires classiquement employés dans la formation du comprimé, de préférence des agents lubrifiants et/ou des agents de désintégration dans lesquels le liquide de granulation est une suspension à 5 à 30% de cellulose microcristalline dont 90% présentent une dimension particulaire inférieure à 50 µm, et la quantité de cellulose microcristalline étant rapportée à la masse de comprimés finale dans l'eau, et/ou l'ethanol et/ou l'isopropanol, ladite suspension pouvant contenir également sous forme dissoute une partie des ingrédients dont sont formés les granules.

2. Un procédé selon la revendication 1, dans lequel 90% de la cellulose microcristalline suspendue dans le liquide de granulation présente une dimension particulaire qui est inférieure à 25 µm.

3. Un procédé selon la revendication 1 ou 2, dans laquelle le liquide de granulation contient, en plus de la cellulose microcristalline , de la poly(vinyl pyrrolidone), de l'hydroxypropyl methyl-cellulose, de l'hydroxypropyl-cellulose, de l'ethyl-cellulose et/ou de la gelatine en tant qu'agents de liaison sous forme dissoute.

4. Un procédé selon la revendication 1 ou 2, selon lequel le liquide de granulation contient, en plus de la cellulose microcristalline, du lactose, du mannitol et/ou du glucose en tant qu'agent de charge, sous forme dissoute.

5. Comprimés préparés par le procédé selon la revendication 1, présentant une résistance à la rupture élevée et un temps de désintégration court.

6. Comprimés tels que revendiqués dans la revendication 5, présentant une résistance à la rupture supérieure à environ 90 N.

7. Comprimés tels que revendiqués dans la revendication 5, présentant un temps de désintégration de 5 à 9 minutes.
